# EUROPÄISCHE PATENTANMELDUNG

(11) **EP 0 642 759 A1**
(43) Veröffentlichungstag der Anmeldung: **15.03.1995**
(21) Anmeldenummer: 94114349.7
(22) Anmeldetag: 13.09.1994
(51) Int. Cl.: A61B 1/07, G01M 11/00

(54) **Einrichtung zur Transmittanzkontrolle bei optischen Geräten mit Lichtleitmedien, insbesondere bei Endoskopieeinrichtungen**

(30) Priorität: 15.09.1993 DE 4331232
(71) Anmelder: Steudel, Hans Werner, D-40764 Langenfeld (DE); Staubes, Doris, D-42699 Solingen (DE)
(72) Erfinder: Steudel, Hans Werner, D-40764 Langenfeld (DE)
(74) Vertreter: Kalisch, Martin

(57) **Zusammenfassung**

Die Erfindung betrifft eine Einrichtung zur Transmittanzkontrolle bei optischen Geräten mit Lichtleitmedien, insbesondere bei Endoskopieeinrichtungen, mit Mitteln zur Erfassung der Beleuchtungsstärke und Umwandlung in eine elektrische Größe, sowie eine Anzeigeeinrichtung zur Visualisierung des Wertes der elektrischen Größe. Um bei einer Einrichtung dieser Art eine Fehlerlokalisierung im gesamten lichtleitenden System samt Lichtquelle und Okular eindeutig, schnell, und mit konstruktiv einfachen und quasi ausfallsicheren Mitteln zu ermöglichen, ist erfindungsgemäß vorgeschlagen, daß als Mittel zur Erfassung der Beleuchtungsstärke ein Lichtsensor (13) vorgesehen ist, welcher in einer einseitig offenen Hülse (10) angeordnet ist, daß Adapter (20,30,40,50) vorgesehen sind, welche auf oder in die Hülse (10) steckbar sind, und daß die Abmessungen der Hülse (10) und der Adapter (20,30,40,50) im Steckbereich so gewählt sind, daß sich eine Spielpassung im Rahmen eines Gleitsitzes ergibt, und daß Hülse (10) und Adapter (20,30,40,50) mit definierten Distanzmitteln (11,31,41,51) versehen sind.

## Beschreibung

Die Erfindung betrifft eine Einrichtung zur Transmittanzkontrolle bei optischen Geräten mit Lichtleitmedien, insbesondere bei Endoskopieeinrichtungen, gemäß Oberbegriff des Patentanspruches 1.

Bei optischen Geräten, m denen Lichtleitmedien verwendet werden, wie bspw. bei Endoskopen, wird das Licht an einem zumeist stationären Ort erzeugt und über eine oder mehrere Lichtleiterstrecken geführt. Das freie Ende des Lichtleiters ist mit einem ebenfalls lichtleitenden Geräteelement zu verbinden, welches im Falle eines Endoskopes die einzuführende Sonde sowie das Beobachtungsokular enthält. Das Beobachtungsokular ist mit einer Kamera verbindbar, die das Endoskopbild aufnimmt und auf einem Monitor sichtbar macht. Es ist physikalisch vorgegeben, daß die an der stationären Lichtquelle anliegende Beleuchtungsstärke nicht verlustfrei bis zum Okular geführt werden kann. Grund dafür ist die materialspezifische optische Extinktion. Als Lichtleitmedien dienen vielfach Lichtleitfasern oder in Kabel eingehüllte Fluide. Die Tansmittanz dieser Lichleitmedien ist Alterungen unterworfen. So können Lichtleitfasern partiell Brüche erleiden und Fluide bspw. aufgrund ihrer zumeist leicht hygroskopischen Eigenschaft altern.

Dies wiederum führt zu einer Reduzierung der Lichtausbeute am Zielort. Zur sicheren Anwendung der Endoskopie bedarf es jedoch des sicheren Betriebes aller Komponenten. Für den Fehlerfall heißt dies, daß der Fehler schnell und treffsicher auffindbar sein muß.

Dies gilt für Endoskope im technischen Anwendungsfall, viel mehr jedoch für Endoskope im medizinischen Anwendungsfall. Von vehementer Wichtigkeit ist eine schnelle Fehlerortung im chirurgischen Anwendungsfall.
Aus der DE 39 29 562 ist eine Einrichtung bekannt, die im Bereich der Lichtquelle einen Strahlenteiler aufweist, über welchen ein Teil des Lichtes abzweigbar und einer Erfassungseinrichtung zuführbar ist. Die am distalen Ende des Lichtleiterkabels austretende Lichtmenge wird über eine zweite Erfassungseinrichtung, an welche das Lichtleitkabel anschließbar ist, gemessen. Eine Signalverarbeitung d.h. eine Rechenschaltung ermittelt den Quotienten aus den erfaßten Lichtmengen. Mit dieser Einrichtung ist die Lichtquelle und das Lichtleitkabel prüfbar.

Bei Endoskopen enthält die gesamte optische bzw. lichtleitende Strecke jedoch nicht nur Lichtleitkabel, sondern umfaßt optische Geräte bzw. Geräteelemente, die ihrerseits mit weiteren lichtleitenden Medien versehen sind, die das Licht bis zum Zielort passieren maß. In den allermeisten Fällen kommt hinzu, daß die Geräte bzw. die Geräteelemente von Betriebsfall zu Betriebsfall, und im chirurgischen Anwendungsfall sogar während eines einzigen Betriebes, gewechselt werden müssen.
So hängt die Wahl der einzuführenden Sonde, die in der genannten lichtleitenden Strecke enthalten ist, vom chirurgischen Einsatzfall ab. D.h. z.B. eine zur Laparoskopie benötigte Sonde ist in der Regel dicker und länger als eine Sonde zur Blasenspiegelung.
Eine schnelle Unterscheidbarkeit, welche der in der gesamten Lichtstrecke vorhandenen Komponenten defekt ist, und somit ausgetauscht werden muß, um bspw. die Operation alsbald weiterführen zu können, ist von entscheidender Bedeutung. Eine On-line Überwachung aller Komponenten d.h auch der einzuführenden Sonde scheidet insbesondere bei der chirurgischen und diagnostischen Anwendung aus, da die Unterbringung von zusätzlichen Prüf-Lichtleitkabeln und Strahlenteilern eine konstruktive Verdickung der Sonde herbeiführen würde.
Dies wiederum würde bei den genannten chirurgischen bzw. diagnostischen Anwendungsfällen eine erheblich höhere Belastung des Patienten darstellen. Da auch während einer Operation die Sonde bzw. das Okular blind werden kann, muß ein solcher Defekt von einem Defekt am Lichtleiterkabelsystem eindeutig unterscheidbar und lokalisierbar sein, um dem Arzt die rasche Behebung des Schadens zu ermöglichen. Dies ist mit dieser bekannten Einrichtung nachteiligerweise nicht möglich. Die Überwachung des dem Lichtleitkabel nachgeordneten, ebenfalls lichtleitenden optischen Geräteelementes ist aus vorstehend genannten Gründen nicht möglich.

Der Erfindung liegt daher die Aufgabe zugrunde, eine Einrichtung der gattungsgemäßen Art derart weiterzubilden, daß eine Fehlerlokalisierung im gesamten lichtleitenden System samt Lichtquelle und Okular eindeutig und schnell, mit konstruktiv einfachen und quasi ausfallsicheren Mitteln möglich ist.

Die gestellte Aufgabe wird bei einer Einrichtung der gattungsgemäßen Art erfindungsgemäß durch die kennzeichnenden Merkmale des Patentanspruches 1 gelöst. Weitere vorteilhafte Ausgestaltungen sind in den Unteransprüchen angegeben.

Im Hinblick auf die eingangs dargestellte Anforderung an die Zuverlässigkeit eines Endoskopes und die Beräcksichtigung des bekannten Standes der Technik, besteht der Kern der Erfindung darin, daß der Lichtsensor in einer einseitig offenen Hülse angeordnet ist und mit einem geeigneten Adaptersystem in Kontakt bringbar ist, bei dem jeder Adapter die Grundmerkmale des Patentanspruches 1 aufweist. Neben diesen konstruktiven Grundmerkmalen weist jeder Adapter für den eigenen spezifischen Meß- bzw. Kontrollort noch weitere eigene Konstruktionsmerkmale auf, die in den Unteransprüchen niedergelegt sind.

Eine möglichst verlustfreie optische Ankopplung zweier oder mehrer Lichtleiter wird bei der vorliegenden Erfindung bewußt vermieden, da eine solche Ankopplung unter der problembedingten Forderung der schnellen Wechselbarkeit viel zu aufwendig und im diagnostischen Fall gar nicht möglich wäre.
Aus diesen Gründen entfernt sich die erfindungsgemäße Sensor- und Adapterkonstruktion bzw. die erfindungsgemäßen Adapterkonstruktionen in der aufgabengemäßen Forderung von einer Lichtleiterverbindung gemäß DE-PS 32 16 560. Die Erfindung vereint in ihrer einfachen Gestaltung mechanische und optische Funktionen in vorteilhafter Art.

Bei der oben genannten bekannten Lichtleiterverbindung soll eine möglichst lichtverlustfreie Ankopplung von Lichtleitfasern untereinander oder einer Lichtleitfaser mit einer Lichtquelle oder einem Detektor erzielt werden. Die vorliegende Erfindung dagegen nimmt eine verlustbehaftete optische Ankopplung an die lichtleitenden Elemente in Kauf, und gleicht dieses Defizit durch Mittel aus, die eine hohe Reproduzierbarkeit und damit hohe Aussagekraft bewirken. Dies wird mit den erfindungsgemäß vorgegeben konstruktiven Mitteln erreicht. Zunächst einmal ist es wesentlich, den Lichtsensor in einer Hülse anzuordnen, so daß der Fremdlichteinfluß nach Aufstecken eines Adapters mitsamt dem auf Transmittanz zu prüfenden lichtleitenden Element, ausgeschlossen ist. Adapter und Hülse weisen dabei zueinander an den sich beim bzw. nach Aufstecken berührenden Aussen- und Innenmantelflächen derartige relative Abmessungen d.h. Fertigungstoleranzen auf, daß sich jeweils Spielpassungen im Rahmen eines Gleitsitzes ergeben, wodurch eine Leichtgängigkeit der Verbindung zwischen Adapter und Lichtsensorhülse besteht, so daß die oben genannte Forderung der schnellen Wechselbarkeit gegeben ist. Die Gleitpassung bzw. der Gleitsitz (nach DIN) ist eine Unterform der Spielpassung und bedeutet, daß die Teile von Hand fügbar bzw. verschiebbar sind. Spezielle Ausgestaltungen und Beispiele für die verwendbaren Spielpassungen sind weiter unten noch angegeben.

Das Merkmal der Spiel- bzw Gleitpassung in Verbindung mit der Anordnung von Distanzmitteln, beispw. in Form von Abstandsringen, führt zu einer reproduzierbaren Abstandseinstellung zwischen Sensor und zu prüfender optischer Schnitt- bzw. Trennfläche. Gleichzeitig ergibt sich durch diese Merkmalkombination eine quasi vollständige Fremdlichtabschirmung, wie durch Versuche bestätigt werden konnte. Da man auf eine direkte lückenfreie optische Ankopplung verzichtet und statt dessen einen Abstand zum Lichtsensor einstellt, muß dieser für jede zu prüfende optische Schnittstelle bei dem entsprechend zu verwendenden Adapter absolut reproduzierbar sein. Die Adapter müssen dabei an jeder beliebigen möglichen Meßstelle im Lichtleitsystem den notwendigen Abstand zum Lichtsensor einstellen und dabei leichtgängig aufsteckbar sein.
Gleichzeitig muß die Steckverbindung zur Vermeindung von Fremdlichteinflüssen weitgehend lichtdicht sein, derart daß Fremdlicht durch Reflexion vom Lichtsensor ferngehalten wird. Alle diese Forderungen sind durch die Kombination der Merkmale des Patentanspruches 1 in überraschend wirkungsvoller Weise gegeben. Die integrierten Abstandsringe haben neben der mechanischen Abstandseinstellung auch optische Fremdlichtabschirmungsfunktion, indem sich das Fremdlicht durch Mehrfach-Reflexion in der im Meßfall gegebenen Steckverbindung derart totläuft, daß es nicht zum Sensor gelangt. Der reproduzierbar einstellbare Abstand des über den Adapter in die Lichtsensorhülse einzuführenden Endes des Lichtleitkabels oder des Okulars oder anderer lichtleitender Elemente des Systems gewährleistet, daß unter Ausschaltung von Fremdlichteinflüßen die Messung des Lichtwertes reproduzierbar aussagekräftig wird. Im medizinischen Anwendungsfall sind dem Arzt die Trausmittanzwerte der einzelnen optischen Elemente bekannt.

Bei einer Fehlfunktion des Endoskopes kann man über die angegebenen verschiedenen Adapterelemente, die in sich wiederum alle Grundelemente des Patentanspruches 1 enthalten, jede Meßstelle des Lichtleitsystems eines Endoskopes durch einfaches Aufstecken und Ablesen der Sensorwerte überprüfen.

Für den medizinischen Anwendungsfall gibt es einen weiteren wesentlichen Vorteil der Erfindung, der darin besteht, daß durch die einfache Konstruktion der Adapterelemente sowie der mit der einzuführenden Endoskopsonde in Berührung zu bringenden Elemente wie Lichtsensorhülse und Adapter eine leichte und zuverlässige Sterilisierbarkeit gegeben ist. Diese Problematik der leichten und zuverlässigen Sterilisierbarkeit gab die in der Aufgabe der Erfindung dargelegte Forderung vor, daß die Mittel konstruktiv einfach ausgebildet sein müssen. Die erfindungsgemäße Lösung, das heißt die erfindungungsgemäße Kombination der Merkmale, erzielt eine Gesamtwirkung, die über die Summe der durch die Merkmale vorgegebenen Einzelwirkungen weit hinausgeht.

In Labortests haben sich die vorstehend genannten Vorteile bestätigt, und es zeigt sich zudem eine sehr einfache Handhabbarkeit der Einrichtung, die insbesondere im chirurgischen Anwendungsfall wichtig ist.

Unter dem Aspekt der Zuverlässigkeit der Einrichtung zeigt es sich als erheblich vorteilhaft, auf die Gesamtfunktion der Einrichtung auch den Betätigungsschalter der dem Lichtsensor nachgeordneten Auzeigeelektronik erfindungsgemäß abzustimmen, derart, daß die Einschalt- bzw.Ausschaltfunktion des Anzeigegerätes funktional auf den Schalter zur Wahl des Batterietestes gelegt ist. Hierbei kommt es im Gegensatz zu bekannten Einrichtungen auf die Reihenfolge der Schaltfunktionen an. Beim Einschalten des Gerätes wird zwangsläufig ein erster Anzeigestatus gewählt, bei dem ein Batterieladetest erfolgt und die verfügbare Klemmenspannung angezeigt wird. Die danach einstellbaren Schaltstellungen betreffen die Einstellung zweier unterschiedlicher Empfindlichkeitsbereiche der Anzeige des Lichtsensors. In der Ausführung als einrastender Drehschalter ist die Reihenfolge der Funktionen in erfindungsgemäßer Weise baulich vorgegeben; das heißt nach Einschalten des Gerätes erfolgt zwangsläufig zuerst ein Batterietest, bevor die eigentlichen Messbereiche aufgeschaltet werden können. Dies ist ein weiteres Element in erfindungsgemäßer Lösung der Aufgabe.

Durch diesen quasi zwanghaft vorgenommenen Batterietest, wird die Fehlerquelle "Elektronik" zunächst überprüft, um eine Fehlerhaftigkeit der Prüfelektronik bei weiterer Fehlersuche ausschließen zu können.
Es besteht also funktionsmäßig ein enges Zusammenwirken der durch die Adapter eingestellten optischen Verhältnisse und der am Anzeigegerät ablesbaren Werte. Dies ist wichtig zur bestimmungsgemäßen Lokalisierung einer Fehlfunktion des zu prüfenden Endoskopes.

In möglicher Ausgestaltung der Erfindung können eine Anzahl der häufigst benötigten Adapter innerhalb einer drehbaren Revolveranordnung oder in einem Adapterhalter fixiert sein. Die Revolveranordnung weist dabei ein Steckelement auf, welches in die Lichtsensorhülse einsteckbar ist, und trägt eine gewisse Anzahl von Adaptern, die auf die jeweiligen zu prüfenden Meßstellen im gesamten Lichtleitweg des Endoskopes angepaßt sind. In den für eine entsprechende Meßstelle vorgesehenen Adapter ist das zu prüfende Lichtleitelement einzustecken, wobei der Adapterrevolver dann soweit zu drehen ist, bis der gewählte Adapter mit der Lichtsensorhülse axial fluchtet.

Für den Test des Endoskopokulares ist ein erfindungsgemäßer Adapter vorgegeben, welcher wiederum auf das Gesamtsystem kompatibel ist. Hierbei sind jedoch spezifische Gestaltungsmerkmale gegeben, die einen Licht-bzw. Transmittanztest am Okular ermöglichen. Die dabei erfindungsgemäß vorgegebenen Gestaltungsmerkmale haben den Effekt, das Okular auf dem Lichtwege zum Lichtsensor von Fremdlichteinflüssen abzukoppeln. Hierbei ist die etwas eigenwillige Außenkontur von gängigen Endoskopokularen mitberücksichtigt. An dem Ende, an dem der Okularadapter aufs Okular aufgesteckt wird, ergibt die im Adapterinnenmantelbereich erfindungsgemäß gegebene Hinterschneidung oder der Innenkonus, daß der Adapter sicher auf das Okukar aufsteckbar d.h. aufklipsbar ist, ohne sich ungewollt zu lösen. Schon aus diesem Grund kann der Okularadapter nicht aus einem unelastischen Werkstoff wie Stahl gefertigt werden. Die weitere erfindungsgemäße konstruktive Ausgestaltung dieses Adapters führt neben der Aufklipsbarkeit auf das Okular zu einer Zentrierung des Lichtsensors auf die vorgegebene optische Achse zum Okular.

Die mögliche Anordnung einer Sammellinse im Bereich der Durchgangsöffnung des Okularadapters macht die Lichtmessung im Okularbereich genauer, da das aus dem Okular austretende Licht vollständig auf den Sensor abgebildet wird. Dies ist deshalb vorteilhaft, da schon die fehlerfreie Lichtausbeute am Okular erheblich geringer ist als an der Lichtquelle oder am Austritt des Lichtleiterkabels.

Die Materialwahl des Polyacetal-Kunststoffes erfüllt dabei sowohl eine mechanische als auch eine optische Funktion. Die mechanische Funktion ist die, daß der Kunststoff elastisch ist, so daß die Hinterschneidung bzw. der Innenkonus des Okularadapters überhaupt auf das Okular aufschiebbar bzw. aufklipsbar ist.
Gleichzeitig bewirkt der Polyacetal-Kunststoff durch seine spezifische Oberfläche und seine wahlweise schwarze Farbe eine optimale Fremdlichtabschirmung, die deshalb unerläßlich ist, weil am Okular -wie oben bereits gesagt- nur noch relativ kleine Lichtmengen vorhanden sind. Das Okular nimmt im Betrieb nämlich nur noch das Licht entgegen, welches am Operationsort reflektiert und wieder in die Endoskopsonde als Bild eingespeist wird.

Zur mechanischen und optischen Funktion kommt bei der Wahl des Polyacetal-Kunststoffes noch eine wesentliche, im medizinischen Anwendungsfall wichtige Eigenschaft hinzu.
Der Polyacetal-Kunststoff erweist sich als mechanisch und materialeigenschaftlich ausgesprochen widerstandsfähig gegen alle üblichen Sterilisierverfahren.
Das heißt der benannte Kunststoff übersteht eine Gammastrahlensterilisation genauso gut wie eine Naßsterilisation im Hochdruckautoklaven. Das heißt der genannte Kunststoff bleibt bei Gammabestrahlung und/oder Erhitzung unter gleichzeitiger Druckanwendung material- und formstabil. Dies ist wegen der beschriebenen Funktion für den Okularadapter von erheblicher Bedeutung.

Vorteilhaft ergibt sich auch, daß die erfindungsgemäß ausgestalteten Adapter bezogen auf ihre axiale Richtung rotationssymmetrisch sind, und somit als Drehteile gefertigt werden können.

Auf diese Weise können die in vorteilhafter Ausgestaltung der Erfindung vorgegebenen Abstandsringe einstückig in den Adapter eingedreht werden. Ebenfalls ist die recht aufwendige Innenkonturierung des Okularadapters einfach durch spanende Bearbeitung herstellbar. Wichtig ist noch zu erwähen, daß alle Adapter, insbesondere jedoch der Okularadapter durch seine innen angeformte Hinterschneidung bzw. durch seinen innen angeformten Konus, ein Aufklicken auf das Okular ermöglichen, so daß nach Einführen der Lichtsensorhülse auf der anderen Seite des Adapters und nach Andrücken von Okular und Lichtsensorhülse in den Adapter alle Elemente gemeinsam auf der optischen Achse fluchten. Gleichzeitig ist in diesem Augenblick die optische Verbindung über den Adapter derart geschlossen, daß Fremdlichteinflüße in oben genannter Weise ausgeschlossen sind.

Die Erfindung ist in der Zeichnung detailliert dargestellt und im nachfolgenden näher beschrieben.

Es zeigt:
- Fig.1: Übersichtsanordnung einer Endoskopieeinrichtung
- Fig.2: Sensorhülse mit Lichtsensor im Längsschnitt,sowie Anzeigegerät
- Fig.3: Adapter zum Prüfen des Lichtleitkabels
- Fig.4: Adapter mit Innenkonus, zum Prüfen des Okulars
- Fig.5: Adapter mit Hinterschneidung zum Prüfen des Okulars
- Fig.6: Reflektor

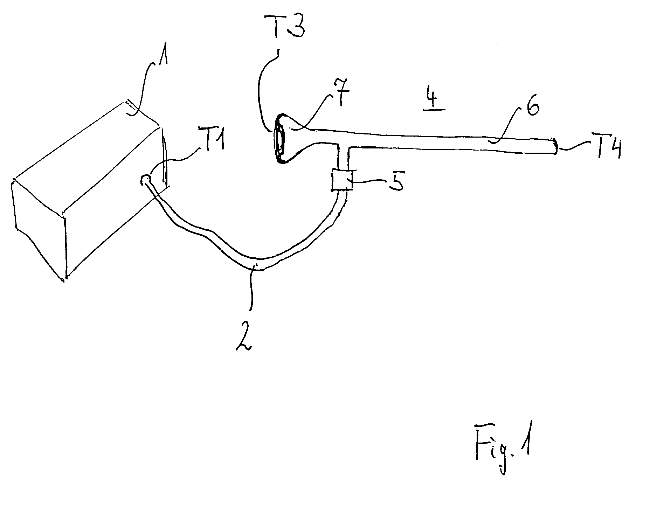

Figur 1 zeigt in Gesamtübersicht eine übliche Endoskopieeinrichtung. In einer regelbaren Lichtquelle 1 ist eine Lampe installiert, welche zumeist über optische Mittel wie Hohlspiegel und/oder Linseneinrichtungen das Licht auf die Trennstelle T1 mehr oder weniger fokussiert, an welche das Lichtleiterkabel 2 anschließbar ist. Das Lichtleiterkabel 2 besteht in der Regel aus gebündelten Lichtfasern. Weniger gebräuchlich, aber dennoch möglich sind auch Lichtleiterkabel, welche mit lichtleitenden Fluiden gefüllt sind. Das distale Ende des Lichtleiterkabels geht an einer Trennstelle T2 in das eigentliche Endoskop 4 über. Das Endoskop 4 verfügt dabei an der Trennstelle T2 über übliche Kopplungsmittel 5 zum möglichst verlustfreien Ankoppeln des Lichtleiterkabels 2 an das Endoskop 4. Innerhalb des Endoskopes 4 ist dabei ein hier nicht dargestellter Lichtumlenker angeordnet, welcher das über das Lichtleiterkabel eingekoppelte Licht zur Austrittsfläche T4 des Endoskopes hinleitet. Die Austrittsfläche T4 ist beim Einsatz des Endoskopes in die zu untersuchende Körperhöhle eingeführt. Über die Lichtaustrittsfläche T4 wird damit die zu untersuchende Körperhöhle beleuchtet, und das von den Objekten der Körperhöhle reflektierte Licht tritt wieder in den rohrförmigen Abschnitt 6 des Endoskopes ein, wobei optische Mittel vorhanden sind, die dann am Okular 7 ein Bild erzeugen.

Die Bild- bzw. Lichtaustrittsfläche am Okular 7 ist im Betrieb eines herkömmlichen Endoskopes üblicherweise mit einer Kamera verbunden, die das Bild weiterverarbeitet und an einem Monitor sichtbar macht. Da die Lichtmenge des im Okular 7 erzeugten Bildes an die Kamera weitergegeben wird, befindet sich auch hier eine Trennstelle T3 des gesamten Lichtweges, die überprüftbar ist.

Die nachfolgend beschriebenen Adapter sind bezüglich der in Fig. 1 im Lichtwege auftrennbaren Trennstellen in ihrer konstruktiven Ausbildung jeweils funktionsspezifische meßstellen-, das heißt trennstellenspezifische Unikate. Dies hat für die medizinische Anwendung den erheblichen Vorteil, daß die Adapter unvertauschbar sind, und somit jene Fehlerquelle ausgeschaltet ist, die darauf beruhen könnte, daß an einer gegebenen Trennstelle ein falscher Adapter benutzt wird, und damit die ansonsten bei richtiger Verwendung reproduzierbaren Abstände zum Sensor nicht mehr gegeben wären.

Die nachfolgend beschriebenen, an den Steckflächen vorgesehenen Abmessungen im Maße einer Spielpassung haben den Vorteil, daß keine mechanische Verriegelung notwendig ist, und trotzdem eine schlüssige Verbindbarkeit und leichtgängige Wechselbarkeit gegeben ist. Dies ist im chirugischen Anwendungsfall besonders wichtig.

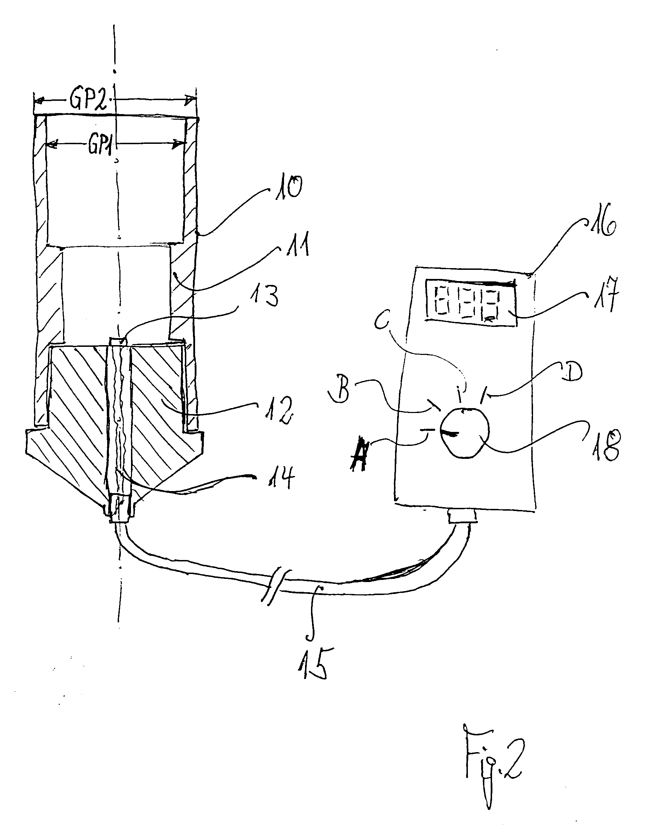

Fig.2 zeigt die Sensorhülse 10 innerhalb derer ein einstuckig angeformte Abstandsring 11-man könnte dies auch als Absatz bezeichnen- angeordnet ist. An einem Ende der Sensorhülse ist ein Sensorträger 12 eingeschoben oder eingeschraubt, welcher das eigentliche lichtempfindliche Sensorelement 13 enthält. Innerhalb des Sensorträgers verlaufen elektrische Leitungen 14, die in ein Kabel 15 münden, welches aus dem Sensorträger 12 austritt und mit dem Anzeigegerät 16 verbunden ist. Die Sensorhülse weist sowohl im Innenmaß als auch im Außenmaß das in der Zeichnung dargestellte Gleitsitzmaß GP1 bzw. GP2 auf.
Diese Gleitsitzmaße ergeben sich aus der Dimensonierung der in die Sensorhülse 10 einzuschiebenden Adapter derart, daß Sensorhülse und jeweiliger einzuschiebender bzw. aufzuschiebender Adapter im Steckbereich, das heißt bis zum Absatz des Abstandsringes 11 das Innenmaß GP1 und im Außenmaß GP2 enthalten. Diese Gleitpassung ergibt sich dimensionsmäßig aus den nach DIN vorgegebenen Fertigungstoleranzen und Oberflächenbeschaffenheiten. So sind die folgenden Kombinationen nach DIN zur Bewerkstelligung der Spielpassung möglich, und vorteilhaft.

| Paarung bei | |
|---|---|
| Einheitsbohrung | Einheitswelle |
| H7/g6 | G7/h6 |
| H7/f7 | F7/h6 |
| H8/h9 | H8/h9 |
| H7/f7 | F8/h9 |
| Noch größere bzw gröbere Paarungen sind theoretisch möglich, aber unvorteilhaft. | |

Auf die in Fig. 1 dargestellten Trennstellen T1-T3 sind nun nachfolgend beschriebene Adapter aufsteckbar, wobei dann das lichtleitende Element mitsamt Adapter in die Sensorhülse 10 eingeschoben wird. Adapter und Sensorhülse sind dabei insgesamt so aufeinander abgestimmt gestaltet, daß das an der entsprechenden Trennstelle austretende Licht zur optischen Achse des Lichtsensors 13 zentriert ist. Die Sensorhülse weist somit sowohl im Innenmaß als auch im Außenmaß ein Gleitsitzmaß GP1 bzw. GP2 auf, welches auf das Innen- bzw. Außenmaß jedes Adapters angepaßt ist, so daß unabhängig davon, ob der jeweilige Adapter durch seine konstruktiven Abmessungen bedingt in die Sensorhülse eingeschoben oder auf die Sensorhülse aufgeschoben wird, alle Adapter auf bzw. in diese eine Sensorhülse passen. Das auf den Lichtsensor 13 fallende Licht wird innerhalb des Sensors in ein elektrisches lichtmengen-bzw.-intensitätsabhängiges elektrisches Signal umgewandelt, welches über die Leitungen 14 bzw. über das Kabel 15 der elektronischen Anzeigeeinrichtung 16 zugeführt wird.

Auf dem Anzeigedisplay 17 wird diese lichtintensitäts-oder lichtmengen-proportionale elektrische Größe angezeigt. Dies geschieht mit Hilfe von bekannten elektrischen bzw. elektronischen Mitteln. Die Einrichtung 16 verfügt dabei über einen Rastschalter 18, der hier als Drehschalter ausgebildet ist. Dieser Drehschalter rastet in diesem Beispiel in 4 Schaltstellungen ein, die grundsätzlich nur nacheinander schaltbar sind. Die erste dargestellte Schaltstellung A ist der ausgeschaltete spannungslose Zustand des Gerätes. Die erste anwählbare Raststellung ist Raststellung B, die einen Batterietest vornimmt. Die nachfolgenden Raststellungen C und D betreffen unterschiedliche Meßempfindlichkeiten. Diese sind deshalb gewählt, da im Bereich der Trennstellen T1 und T2 deutlich höhere Lichtintensitäten anfallen als an der Trennstelle T3 am Austritt des Okulars 7. Der Rastschalter 18 gibt somit vor, daß um in einen der beiden Meßbereiche schalten zu können, zunächst die Raststellung B für Batterietest überfähren werden muß. Dabei kann die Raststellung B zum Batterietest beispielsweise elektromechanische Verriegelungselemente enthalten, die ein Weiterdrehen des Schalters blockieren, wenn die Batterie nicht mehr die notwendige Klemmenspannung aufweist.

Es wäre auch denkbar, daß im Falle einer mangelhaften Klemmenspannung der Batterie ein Blinken der Anzeige oder ein optisches oder akustisches Warnsignal ausgelöst wird.

Wichtig ist hierbei grundsätzlich, daß die Batterietestfunktion zwanghaft angewählt werden muß, das heißt man kann die Meßbereiche C und D nur dann einstellen, wenn man vorher den Rastschalter über die Schaltstellung B gefahren und somit einen Batterietest eingeleitet bzw. vorgenommen hat.

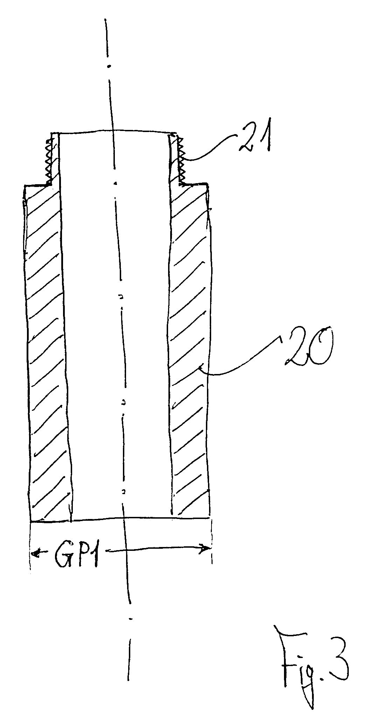

Fig.3 zeigt im Längsschnitt einen Lichtleiteradapter 20, der für die Trennstelle T2 zur Überprüfüng des Lichtleiterkabels 2 aus Fig.1 vorgesehen ist. Der Lichtleiteradapter 20 weist ein offenes Ende auf, in welches das Lichtleiterkabel einführbar ist. Hierfür weist der Adapter Befestigungsmittel 21 auf, über welche das Lichtleiterkabel am Adapter befestigbar ist. Bei Endoskopen sind bei den Lichtleiterverbindungen meistens Schraub- oder Bajonettverbindungen üblich. Die Befestigängsmittel 21 des Adapters 20 sind dann entsprechend ausgebildet. Im Außenmantelbereich des Lichtleiteradapters 20 weist die entsprechende Außenfläche wiederrum das vorgegebene Gleitsitzmaß GP1 auf, welches auf das Innenmaß GP1 der Lichtsensorhülse 10 aus Fig.2 in der vorstehend benannten Weise abgestimmt ist. Hierbei bedeutet GP1 als Außenmaß die Eingruppierung als Einheitswelle, und GP1 als Innenmaß die Eingruppierung als Einheitsbohrung. GP1 gilt hier also für in die Sensorhülse 10 einzuschiebende Adapter; bspw. Adapter 20.

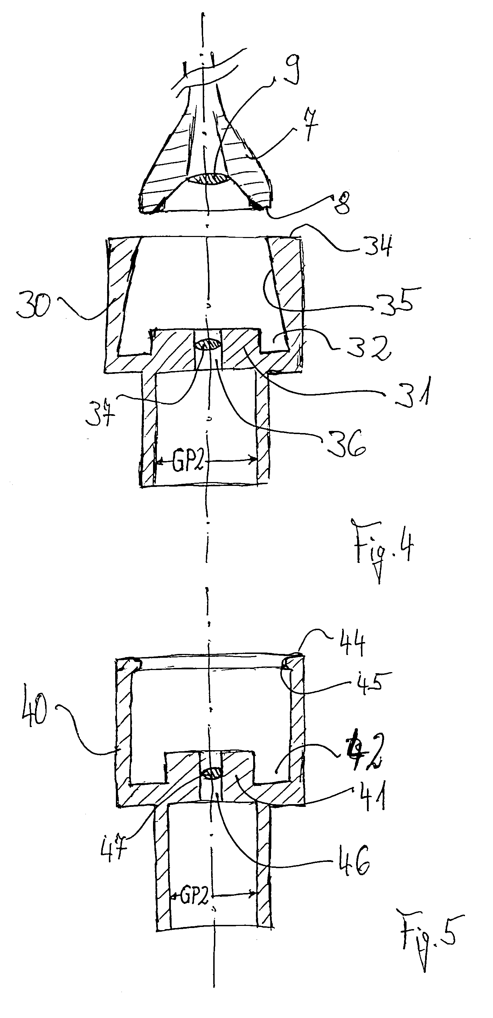

Fig.4 zeigt eine Ausführungsform eines Okularadapters 30. Der Okularadapter 30 ist mit dem offenen Ende 34 auf das Okular 7 des Endoskopes 4 aufsteckbar. Dabei ist das Innenmaß der gebildeten Öffnung an dem Ende 34 des Adapters 30 etwas kleiner als das Anßenmaß des Okulars 7. Nach innen in den Adapter hinein erstreckt sich nun ein Konus 35, welcher sich zum hierbei eigenwillig gestalteten Abstandsring 31 hin erweitert. Dieser Adapter ist dabei aus einem elastischen Kunstoff gefertigt, so daß der Adapter 30 auf das Okular 7 mit leichter Kraftanwendung aufsteckbar ist.

Die Konizität des Innenkonus 35 ist dabei so gewählt, daß sich der Durchmesser dieser Adapteröffnung zum Abstandsring hin mindestens bis auf das Außenmaß des Okulars 7 erweitert. Eine weitere Besonderheit dieses Okularadapters ist, daß der einstückig angeformte Abstandsring 31 an der Seite, welcher dem Okular zugewandt ist, mit einer Hinterdrehung 32 versehen ist. Diese Hinterdrehung ist dabei so dimensoniert, daß der Rand 8 des Okulars 7 in diese Hinterdrehung 32 bei vollständigem Aufstecken des Adapters auf das Okular einlegbar ist. Der Okularadapter ist hierbei im Längsschnitt gezeigt und die Symmetrieachse deutet auf die Rotationsymmetrie dieses Adapters hin. Das heißt mit anderen Worten, daß in den allermeisten Fällen der Adapter im Querschnitt rund ist. Für den Fall, daß beim Endoskop Okulare mit elliptischer Außenkontur verwendet werden, kann der entsprechend auf das Okular aufzusteckende Adapterbereich auch in der Ausgestaltung elliptisch sein. Der untere Abschnitt des Adapters 30 weist ein Innenmaß GP2 auf, so daß er zusammen mit der Sensorhülse 10 in dem Steckbereich d.h. hier im Außenmantel einen Gleitsitz bildet. Dieser Okularadapter wird also auf die Sensorhülse aufgeschoben und nicht eingeschoben. Darauf abgestimmt ist also das Außenmaß der Sensorhülse 10. Somit bildet das Außenmaß der Sensorhülse 10 und das Innermaß des Okularadapters einen Gleitsitz GP2 nach einer der oben angegebenen Paarungen von Einheitswelle und Einheitsbohrung. Der Abstandsring 31 bildet bzw. umgreift eine Durchlaßöffnung 36, welche wahlweise auch mit einer Sammellinse 37 versehen sein kann. Diese Sammellinse 37 muß die durch die Bildfläche 9 des Okulars gebildete Fläche möglichst vollständig auf die Oberfläche des Lichtsensors abbilden, wenn Adapter, Okular und Sensorhülse zur Kontrollmessung zusammengefügt sind. Wichtig ist hierbei, daß die entsprechend angeordneten Distanzmittel d. h. Abstandsringe innerhalb des Adapters, aber auch innerhalb der Sensorhülse, in welche der Adapter einzuführen ist, eine immer wieder reproduzierbare Position der Elemente zueinander gewährleistet, so daß die Messung immer reproduzierbar ist. Eine zusätzliche Linse 37 ist daher vorteilhaft, da die Okularaustrittsfläche 9 auf die Sensoroberfläche 13 abgebildet und somit ganz ausgenutzt wird.

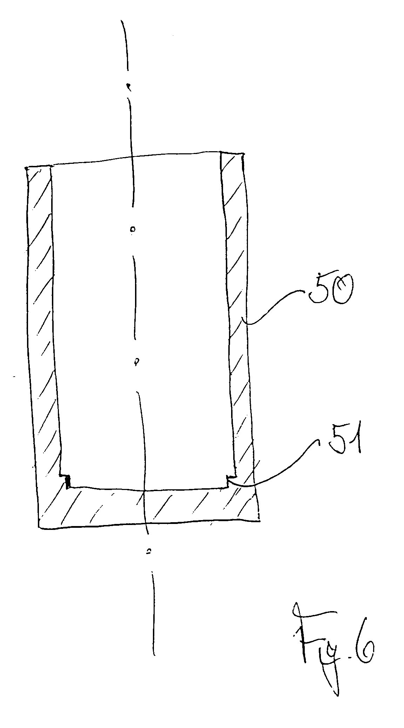

Fig.5 zeigt eine zweite Variante eines Okularadapters, bei der nicht ein Innenkonus 35 wie in Fig.4, sondern eine Hinterschneidung 45 verwendet ist. Diese wirkt im Prinzip im gleichen Maße wie der in Fig.4 angegebene Innenkonus.
Die Hinterschneidung 45 hat dabei eine Durchgangsweite, die ebenfalls etwas kleiner ist als die Außenabmessung des Okulars 7. Durch die Verwendung von elastischem Material ist es möglich, den Adapter 40 mit leichtem Druck auf das Okular 7 aufzuschieben. Die Hinterschneidung verhindert nach Aufstecken auf das Okular ein ungewolltes Lösen bzw. Herunterfallen. Alle übrigen Elemente sind wie die in Fig.4.

Wesentlich an dem Okularadapter nach Fig.4 oder Fig.5 ist, daß der Adapter mitsamt allen Konturen und Anformungen einstückig ausgebildet werden kann. Beim Aufstecken des Adapters 30 bzw. 40 auf die Sensorhülse 10 läßt er sich soweit aufschieben, bis die Sensorhülse am Abstandsring 31 bzw. 41 anliegt

Fig.6 zeigt für den Fall, daß die Trennstelle T3, das heißt das Okular getestet wird, einen auf die Austrittsfläche T4 des Endoskopes 4 aufsteckbaren Reflektor. Hierbei ist auch der Reflektor quasi als Rohrabschnitt also hülsenförmig ausgebildet. Er weist ein offenes Ende und einen geschlossenen Boden auf. Der geschlossene Boden kann entweder durch eine einstückige, das heißt becherförmige Ausgestaltung des Reflektors erfolgen; der Boden kann jedoch auch durch einen Stopfen verschlossen sein. Wesentlich ist auch hierbei, daß der Reflektor einen Abstandsring 51 aufweist, welcher einen von Null verschiedenen, jedoch hinreichend kleinen Abstand der Austrittsfläche T4 des Endoskopes 4 zum Boden, bzw. zum Stopfen einstellt. Es hat sich bei Tests gezeigt, daß der Abstand zwischen Austrittsfläche T4 und reflektierendem Boden bzw. Stopfen sehr klein, aber unbedingt von 0 verschieden sein muß. Dies führt dann zu einer quasi verlustfreien Reflexion des über die Trennstelle T2 in das Endoskop 4 eingekoppelten Lichtes zum Okular 7 hin.

Mit einem Satz von Adaptern, wie vorstehend beschrieben, kann die gesamte Endoskopieeinrichtung bezüglich der Trennstellen T1-T4 sukzessive getestet werden. Die Adapter ermöglichen somit, daß für alle Trennstellen nur ein Sensor notwendig ist. Die Adapter selbst sind bezüglich ihrer Ausgestaltungen auf die jeweilige Trennstelle des Endoskopes abgestimmt und unvertauschbar. Wesentlich ist, daß die Sensorhülse im Zusammenwirken mit den Adaptern sowohl ein Innen- als auch ein Außenpaßmaß aufweist. Somit sind die Adapter unter Nutzung des erfindungsgemäßen Prinzips und abhängig von ihrer Ausgestaltung ein- bzw. aufsteckbar. Das Wesen der Erfindung zeigt sich hier vorteilhaft im Rahmen von Universalität.
Das Wesen der Erfindung ist in der Technik, wo konstruktive Hohlräume zu untersuchen sind, ebenso vorteilhaft einsetzbar. An der Lichtquelle selbst, d.h. an der Trennstelle T1 läßt sich die Lichtemission der Lichtquelle 1 direkt messen, indem man lichtquellen-spezifische Adapter konstriert, die zusätzlich mit den erfindungsgemäßen Merkmalen ausgestattet sind.

Bei Verwendung des erfindungsgemäßen Prinzipes, nämlich
- Sensor in Hülse
- Hülse mit Innen- und Außenpaßmaß
- Adapter mit Innen- bzw- Außenpaßmaß
- Hülse und Adapter mit Distanzmitteln
ist es möglich, mit Hilfe nur eines einzigen Sensors an jeder beliebigen, durch jeweilige Adapter paßbar gemachten Trennstelle eines Endoskopsystems mit hoher Genauigkeit die Transmittanz zu prüfen.

### Bezugszeichenliste:

- 1: Lichtquelle
- 2: Lichtleiterkabel
- 4: Endkoskop
- 5: Kopplungsmittel
- 6: rohrförmiger Abschnitt
- 7: Endkoskopokular
- 8: Rand des Okulars
- 9: optische Fläche des Endkoskopokulars
- 10: Sensorhülse
- 11,31,41,51: Abstandsring
- 12: Sensorträger
- 13: Sensorelement
- 14: elektr. Leitungen
- 15: Kabel
- 16: Anzeigeeinrichtung
- 17: Anzeigedisplay
- 18: Rastschalter
- 20,30,40,50: Adapter
- 32,42: Hinterdrehung
- 34,44: offenes Ende des Okularadapters
- 35: Innenkonus
- 45: Hinterschneidung
- 36,46: Durchgangsöffnung
- 37,47: optische Linse
- T1-T4: Trennstellen im Lichtweg der Endoskopieeinrichtung
- GP1, GP2: Spielpassung Gleitsitz
- A,B,C,D: Schaltstellungen

## Patentansprüche

1. Einrichtung zur Transmittanzkontrolle bei optischen Geräten mit Lichtleitmedien, insbesondere bei Endoskopieeinrichtungen, mit Mitteln zur Erfassung der Beleuchtungsstärke und Umwandlung in eine elektrische Größe, sowie eine Anzeigeeinrichtung zur Visualisierung des Wertes der elektrischen Größe,
**dadurch gekennzeichnet,**
daß als Mittel zur Erfassung der Beleuchtungsstärke ein Lichtsensor (13)vorgesehen ist, welcher in einer einseitig offenen Hülse (10) angeordnet ist, daß Adapter (20,30,40,50) vorgesehen sind, welche auf oder in die Hülse (10)steckbar sind, und daß die Abmessungen der Hülse (10) und des Adapters (20,30,40,50) im Steckbereich so gewählt sind, daß sich eine Spielpassung im Rahmen eines Gleitsitzes ergibt, und daß Hülse (10) und Adapter (20,30,40,50) mit definierten Distanzmitteln (11,31,41,51) versehen sind.

2. Einrichtung zur Transmittanzkontrolle bei opitschen Geräten mit Lichtleitmedien, nach Anspruch 1,
**dadurch gekennzeichnet**,
daß die Adapter rohrförmig und die Distanzmittel als Abstandsringe (11,31,41,51) ausgebildet sind, und daß die Abstandsringe einstückig in bzw. an Hülse (10) und Adapter(20,30,40,50) angeformt sind

3. Einrichtung zur Transmittanzkontrolle bei optischen Geräten mit Lichtleitmedien, nach Anspruch 2,
**dadurch gekennzeichnet**,
daß ein zum Ankoppeln an ein Lichtleiterkabel (2) eines Endoskopes vorgesehener Adapter an einem Ende mit Kopplungsmitteln (21) zur Aufnahme des Lichtleiterkabels versehen ist.

4. Einrichtung zur Transmittanzkontrolle bei optischen Geräten, nach Anspruch 1 oder Anspruch 2,
**dadurch gekennzeichnet**,
daß ein zum Aufstecken auf ein Endoskopokular (7)vorgesehener Adapter(30) an dem auf das Okular (7) aufsteckbaren offenen Ende (34) im Innenumfang kleiner als der Außenumfang des Endoskopokulars (7)ist, und sich bis zum innerhalb des Adapters (30) angeordneten Abstandsring (31) auf mindestens das Umfangsmaß des Okulars (7) erweitert.

5. Einrichtung zur Transmittanzkontrolle bei optischen Geräten, nach Anspruch 4,
**dadurch gekennzeichnet**,
daß die vom offenen Ende (34) bis zum Abstandsring (31)gegebene Innenumfangsänderung durch einen sich vom offenen Ende (34) zum Abstandsring (31)nach innen erweiternden Konus gebildet ist.

6. Einrichtung zur Transmittanzkontrolle bei optischen Geräten mit Lichtleitmedien, nach Anspruch 4,
**dadurch gekennzeichnet**,
das die Innenumfangsänderung des Okularadapters (40) durch ein wulstförmige Hinterschneidung (45) gebildet ist.

7. Einrichtung zur Transmittanzkontrolle bei optischen Geräten nach Anspruch 5 oder 6,
**dadurch gekennzeichnet**,
daß der Abstandsring (31,41) über einen Teil seiner axialen Erstreckung eine Hinterdrehung (32,42) aufweist, in welche sich nach Aufstecken auf das Endoskopokular (7) der Okularrand (8) einlegen kann.

8. Einrichtung zur Transmittanzkontrolle bei optischen Geräten nach Anspruch 7,
**dadurch gekennzeichnet**,
daß der hinterdrehte einstückige Abstandsring (31,41) des Okularadapters (7) eine axiale Durchgangsöffnung (36,46) bildet, deren Innendurchmesser größer oder gleich dem Innendurchmesser der optischen Fläche (9) des Okulares (7) ist.

9. Einrichtung zur Transmittanzkontrolle bei optischen Geräten, nach Anspruch 8,
**dadurch gekennzeichnet**,
daß innerhalb der Durchgangsöffnung (36,46) eine optische Linse (37,47) angeordnet ist, welche bezüglich ihrer Brennweite auf die durch Adapter (30,40) und steckbare Sensorhülse (10) vorgegebenen Abstandsverhältnisse von zu testender optischer Fläche (9) zum Lichtsensor (13) abgestimmt ist, derart, daß die optische Fläche (9) auf den Sensor (13) abbildbar ist.

10. Einrichtung zur Transmittanzkontrolle bei optischen Geräten nach einem oder mehreren der vorhergehenden Ansprüche 1-8,
**dadurch gekennzeichnet,**
daß die Adapter (20,30,40,...) mitsamt aller Konturen jeweils einstückig ausgebildet sind.

11. Einrichtung zur Transmittanzkontrolle bei optischen Geräten nach einem oder mehrere der vorhergehenden Ansprüche,
**dadurch gekennzeichnet,**
daß zum Transmittanztest des Endoskopes mittels Okularadapter (30, 40) ein zusätzlicher becherförmiger Adapter (50) vorgesehen ist, welcher auf die Austrittsfläche (T4) des Endoskopes aufsteckbar ist, und im Bereich des Bodens einen Abstandsring (51) aufweist.

12. Einrichtung zur Transmittanzkontrolle bei optischen Geräten nach Anspruch 11,
**dadurch gekennzeichnet,**
daß der Boden durch einen einsteckbaren Stopfen gebildet ist.

13. Einrichtung zur Transmittanzkontrolle bei optischen Geräten nach einem oder mehreren der vorhergehenden Ansprüche,
**dadurch gekennzeichnet,**
daß die Adapter (20,30,40,50) aus Polyacetal-Kunststoff bestehen

14. Einrichtung zur Transmittanzkontrolle bei optischen Geräten nach einen oder mehreren der vorhergehenden Ansprüchen,
**dadurch gekennzeichnet**,
daß die elektronische Anzeigeeinrichtung (16)des Sensors über einen Rastschalter (18) verfügt, der von der ausgeschalteten Stellung "0" (A) der Anzeigeeinrichtung (16) betrachtet, über mindestens zwei weitere Raststellungen (B,C...) verfügt, wobei die erste Schaltstellung (B) nach Stellung (A) einen Batterietest, und die zweite und jede weitere Schaltstellung (C,D...)jeweils einen eigenen Anzeigemeßbereich für den Sensor anwählt.

15. Einrichtung zur Transmittanzkontrolle bei optischen Geräten nach Anspruch 14,
**dadurch gekennzeichnet**,
daß die einzelnen Schaltstellungen (A,B,C,D....) nur nacheinander anwählbar sind.

16. Einrichtung zur Transmittanzkontrolle bei optischen Geräten nach Anspruch 15,
**dadurch gekennzeichnet**,
daß der Rastschalter (18) mit elektromechanischen Verriegelungselementen versehen ist, welche bei Feststellung eines mangelhaften Ladezustandes der Batterie ein Weiterdrehen des Rastschalters in den bzw. in die Anzeigemeßbereiche blockiert.

17. Einrichtung zur Transmittanzkontrolle bei optischen Geräten nach Anspruch 15,
**dadurch gekennzeichnet**,
daß der Rastschalter nach Feststellung eines mangelhaften Ladezustandes der Batterie über elektronische Mittel in allen weiterenSchaltstellungen (B,C,D...) außer (A) ein akustisches oder optisches Warnsignal erzeugt.

18. Einrichtung zur Transmittanzkontrolle bei optischen Geräten nach einem oder mehreren der vorhergehenden Ansprüchen,
**dadurch gekennzeichnet**,
daß der Mindest-Soll-Ladezustand der Batterie quantitativ einstellbar ist.
